# EUROPÄISCHE PATENTSCHRIFT

(11) **EP 2 152 369 B1**
(45) Veröffentlichungstag und Bekanntmachung des Hinweises auf die Patenterteilung: **08.08.2012**
(21) Anmeldenummer: 08718366.1
(22) Anmeldetag: 31.03.2008
(51) Int. Cl.: A61K 49/04, A61P 29/00, A61P 35/00, A61K 31/025, A61K 49/18

(54) **VERWENDUNG VON FLUORHALTIGEN VERBINDUNGEN ZU DIAGNOSEZWECKEN MIT HILFE BILDGEBENDER VERFAHREN**
USE OF FLUORIDE-CONTAINING COMPOUNDS FOR DIAGNOSTIC PURPOSES WITH THE HELP OF IMAGING PROCESSES
UTILISATION DE COMPOSÉS CONTENANT DU FLUOR À DES FINS DE DIAGNOSTIC PAR IMAGERIE

(30) Priorität: 29.03.2007 DE 102007015598
(43) Veröffentlichungstag der Anmeldung: 17.02.2010
(73) Patentinhaber: Heinrich-Heine-Universität Düsseldorf, 40225 Düsseldorf (DE)
(72) Erfinder: SCHRADER, Ulrich, 40597 Düsseldorf (DE); FLÖGEL, Ulrich, 40215 Düsseldorf (DE)
(74) Vertreter: Kutzenberger, Helga
(86) Internationale Anmeldenummer: PCT/EP2008/053838
(87) Internationale Veröffentlichungsnummer: WO 2008/119790

(56) Entgegenhaltungen:
- EP-A- 1 088 558
- WO-A-02/13874
- WO-A-02/13875
- WO-A-90/14846
- WO-A-99/01161
- WO-A-2007/009637
- WO-A-2007/009638
- US-A- 4 767 610
- US-A- 5 114 703
- MATTREY R F: "THE POTENTIAL ROLE OF PERFLUOROCHEMICALS (PFCS) IN DIAGNOSTIC IMAGING" ARTIFICIAL CELLS, BLOOD SUBSTITUTES, AND IMMOBILIZATIONBIOTECHNOLOGY, MARCEL DEKKER INC, US, Bd. 22, Nr. 2, 1. Januar 1994 (1994-01-01), Seiten 295-313, XP000939198 ISSN: 1073-1199
- HANNA G ET AL: "Indirect lymphography with perflubron emulsion: Preclinical and clinical results" INVESTIGATIVE RADIOLOGY 1994 US, Bd. 29, Nr. SUPPL. 2, 1994, Seiten S33-S35, XP008095695 ISSN: 0020-9996

## Beschreibung

Gegenstand der vorliegenden Erfindung ist die Verwendung von fluorhaltigen Verbindungen für den diagnostischen Nachweis inflammatorischer, pathologischer Zustände mit Hilfe von MR-Bildgebung und PET.

Entzündliche Erkrankungen sind weltweit mit Abstand die wichtigsten Ursachen für Morbidität und Mortalität. Während für akute Entzündungskrankheiten (überwiegend erregerbedingt) schon vielfach wirksame Diagnose- und Therapiemöglichkeiten bestehen, ist die Diagnose von chronisch entzündlichen Erkrankungen meist schwierig und die Therapie ist auf symptomatische Maßnahmen beschränkt. Nichtinvasive bildgebende Verfahren wie Echokardiographie, Computertomographie und Kernspinresonanz liefern detaillierte anatomische Informationen und sind dadurch wertvolle Hilfsmittel zur Beurteilung von Organfunktionen. Allerdings ist es bis jetzt mit keinem der genannten Verfahren eindeutig möglich, entzündliche Prozesse mit hoher örtlicher Auflösung nachzuweisen.

Arens et al. beschreiben in Nature Biotechnology Vol. 23 Nr. 8, August 2005 p. 983-987 ein Verfahren zum Aufspüren immuntherapeutischer Zellen. Dabei wird mittels Bildgebung durch magnetische Kernresonanz (MRI) von Perfluoro-15-kronen-5-ether versucht, *in vivo* Zellen zu detektieren, die immuntherapeutische Relevanz besitzen. Verwendet wurden dendritische Zellen, die *ex vivo* mit Perfluro-15-kronen-5-ether beladen wurden. Deren Verbleib nach Injektion in Mäusegewebe oder intravenös verabreicht an Mäusen wurde anschließend *in vivo* untersucht. Ähnliche Untersuchungen sind auch Gegenstand der WO 2005/072780 A2 sowie WO 03/075747 A2 gewesen. G.M. Lanza et al. beschreiben in Medica Mundi 47/1 April 2003, p. 34-39 den Einsatz von paramagnetischen Nanopartikeln für ein Targeting von Zellen. Dabei werden die paramagnetischen Nanopartikel eingesetzt, um Signalauslöschungen im ¹H-Magnetresonanzbild zu erzeugen. Als Sonde werden mit ¹⁹F-beladene Vehikel eingesetzt.

Die DE 694 33 723 T2 betrifft ein Verfahren für die in-vitro Verabreichung von biologischen Substanzen und hierfür verwendbare Zusammensetzungen. Dabei werden die biologischen Substanzen in Verbindung mit einer polymeren Hülle verabreicht.

Die WO-A-2005/072780 betrifft die *ex vivo* Markierung von isolierten Zellen mit Perfluorcarbonen. Es wird offenbart, dass isolierte Zellen, die *ex vivo* mit Perfluorkarbonen markiert worden sind, nach Injektion zum Studium von Zellmigrationen im Organismus geeignet sind.

Die DE-A-41 27 442 betrifft eine Technik zur Herstellung perfluorcarbonhaltiger Liposomen für den Sauerstofftransport, das heißt als Blutersatzmittel.

Maeda, Noriyuki, et al. offenbaren in Biol. Pharm. , Bull.29, 9, 2006, S. 1936 - 1940 ¹⁸Fluor-Deoxiglukose, die in Liposomen verkapselt sind, zusammen mit der PET, um die Ablagerung von Liposomen in Tumorgewebe in-vivo zu untersuchen.

Oku, Naoto, et al. (Biochimica et Biophysics Acta 1280 (1996), 149 - 154) betrifft die Verwendung von Liposomen, die mit ¹⁸F-markierte Glukose geladen wurden, zur Untersuchung von "liposomalem trafficking". Die Untersuchungen wurden mittels PET-Diagnostik durchgeführt.

US 5114703 betrifft den diagnostischen Nachweis von krebs mit einem bildgebendem Verfahren

Aufgabe der vorliegenden Erfindung war es, ein Verfahren bereitzustellen, mit dem es gelingt, spezifisch pathologische Zustände, beispielsweise entzündliche Prozeße, mit Hilfe bildgebender Verfahren darzustellen und die betroffenen Gebiete somit zu erkennen.

Gelöst wird das der Erfindung zugrundeliegende technische Problem durch die Verwendung von fluorhaltigen Verbindungen gemäß dem Ansprüchen für den diagnostischen Nachweis entzündlicher Vorgänge mittels eines bildgebenden Verfahrens, wobei entzündliche Prozeße ausgewählt sind aus der Gruppe bestehend aus pathologischen Vorgängen im Randbereich von Infarkten, wie Herzinfarkt, Schlaganfall, Tumoren; Entzündung von Organen wie Myokarditis, Encephalitis, Meningitis (Hirn und Rückenmarkshäute); Multiple Sklerose; Entzündungen des Magen-Darm-Traktes wie Morbus Crohn; Entzündung der Gefäße, wie Arteriosklerose insbesondere sog. "vulnerable plaques"; Detektion von Abszessen sowie Arthritis. Außerdem können Lymphknoten dargestellt werden und deren pathologische Veränderungen insbesondere bei entzündlichen Vorgängen des Lymphsystems, wie Krebsarten, die direkt die Lymphknoten befallen, insbesondere Morbus Hodgkin, Non-Hodgkin-Lymphome, Tumor-absiedlungen z.B. bei Brustkrebs. Als bildgebendes Verfahren kommt die magnetische Kernresonanzmessung des ¹⁹F-Isotops in Frage. Die Auswertung der entsprechenden Messungen und Umsetzung in ein Bild sind dem Fachmann an sich bekannt, wie sich z.B. aus

Haacke ME, Brown WR, Thompson MR, Venkasetan R: Magnetic Resonance Imaging - Physical Principles and Sequence Design, Wiley, New York, 1999; Yu JX, Kodibagkar VD, Cui W, Mason RP. 19F: a versatile reporter for noninvasive physiology and pharmacology using magnetic resonance; Curr Med Chem. 12:819-48, 2005;
Wernick MN, Aarsvold JN Emission Tomography: The Fundamentals of PET and SPECT, Academic Press, London, 2004 ergibt.

Die im Journal of the American College of Cardiology von W. Gregory Hundley et al. veröffentlichte Methode der intravenösen Verabreichung eines Perfluorocarbonkontrastmittels zur Verbesserung der echo-kardiographischen Bestimmung des linken Ventrikularvolumens und Ejektionsfraktion (JACC, Vol. 32, No. 5, (1981): 1426-32) ist vom Gegenstand der vorliegenden Erfindung nicht erfasst.

Die Erfindung beruht auf der Erkenntnis, dass perfluorierte Verbindungen, wie Fluorcarbone, von Moncyten/Macrophagen in solcher Weise aufgenommen werden, dass eine spezifische Markierung dieser Zellen erfolgt, die in bildgebenden Verfahren diagnostisch genutzt werden kann. Überraschenderweise zeigte sich dabei ein diagnostisches Potenzial zur Sichtbarmachung von entzündlichen Vorgängen und Lymphknoten.

Die fluorhaltigen Verbindungenn liegen in einem Vehikel vor. Als Vehikel kommen , Liposomen in Frage.

Die Liposomen können insbesondere unilamellare Liposomen sein, die der Fachmann in an sich bekannter Weise herstellen und mit fluorhaltigen Verbindungen beladen kann. Dabei ist es nötig, dass die Liposomen in einer Größe appliziert werden, die für die Aufnahme durch immunkompetente Zellen (insbesondere Makrophagen) geeignet ist, in einer Größe zwischen 100-200 nm. Auch andere Liposomenstrukturen als die genannten unilamellaren kommen in Frage. Liposomen und deren Herstellung sind Gegenstand verschiedener Publikationen und dem Fachmann wohlbekannt (Mozafari MR, Liposomes: an overview of manufacturing techniques. Cell Mol Biol Lett 10 (2005) 711-9; Basu SC, Basu M, Methods in Molecular Biology: Liposomes Methods and Protocols Humana Press Inc., Totowa, NJ, 2002).

Die fluorhaltigen Verbindungen sind ausgewählt aus der Gruppe bestehend aus organischen perfluorierten Verbindungen, nämlich

In Perfluoroctylbromid, Die Anwesenheit des ¹⁹F-Isotops in den fluorierten Verbindungen erlaubt den Vorteil des Einsatzes von in der Klinik bereits bekannten und vorhandenen Geräten, nämlich der magnetischen Kernresonanzspektroskopie mit ¹⁹F-Isotopen

Durch die erfindungsgemäße Verwendung fluorhaltiger Verbindungen, insbesondere bei Applikation im Vehikel lassen sich die folgenden pathologischen Zustände nachweisen:
1) Darstellung von Lymphknoten und deren pathologische Vergrößerung
   a) Krebsarten, die direkt die Lymphknoten befallen : Morbus Hodgkin, Non-Hodgkin-Lymphome;
   b) Tumorabsiedlungen z.B. bei Brustkrebs;
   c) Virale und bakterielle Infektionen z.B Syphilis, Tuberkulose;
2) Entzündungsreaktionen im Randbereich von
   a) Infarkten z.B. Herzinfarkt, Schlaganfall;
   b) Tumoren;
3) Entzündung von Organen: Myokarditis, Encephalitis, Meningitis (Hirn und Rückenmarkshäute);
4) Multiple Sklerose;
5) Entzündungen des Magen-Darm-Traktes z.B. Morbus Crohn;
6) Entzündung der Gefäße, z.B. Arteriosklerose insbesondere sog. "vulnerable plaques";
7) Detektion von Abszessen.
8) Nachweis von Arthritis

Die Erfindung wird im Folgenden beispielhaft näher erläutert:

Die Detektion lokaler Entzündungsprozesse mittels MR-Bildgebung (MRI) gelang bisher lediglich unter Verwendung superparamagnetischer Eisenoxidpartikel (SPIOs), wobei man sich die hohe Affinität dieser Teilchen für das Monozyten-Makrophagen-System zunutze macht. Da SPIOs zu einer Auslöschung des MR-Signals führen, sind die Daten in manchen Fällen nur schwierig zu interpretieren, da keine klare Unterscheidung von anderweitigen Artefakten möglich ist. Ziel dieser Arbeit war es daher, ein MR-Verfahren zu etablieren, mit dem entzündliche Prozesse erstmals mit einem positiven Kontrast dargestellt werden können. Als Kontrastmittel wurden emulgierte Perfluorcarbone (PFCs) eingesetzt, die biochemisch inert sind und ebenso wie SPIOs vom Monozyten-Makrophagen-System phagozytiert werden.

Zur Auslösung akuter Entzündungsprozessese wurden zwei verschiedene Verletzungsmodelle (Herz- und Hirninfarkt) in der Maus verwendet. Der Myokardinfarkt wurde durch Ligatur der linken Koronararterie (LAD) ausgelöst. In einer separaten experimentellen Serie wurde das Photothrombosemodell zur Induktion einer fokalen Ischämie im Hirn verwendet. Zu verschiedenen Zeitpunkten nach den genannten Eingriffen wurden 100-500 µl einer 10%igen Emulsion des Perfluoro-15-kronen-5-ether (15C5) in die Schwanzvene der Maus injiziert. Zum histologischen Nachweis der PFCs wurde in einigen Experimenten alternativ eine Rhodamin-markierte 15C5-Emulsion verwendet.

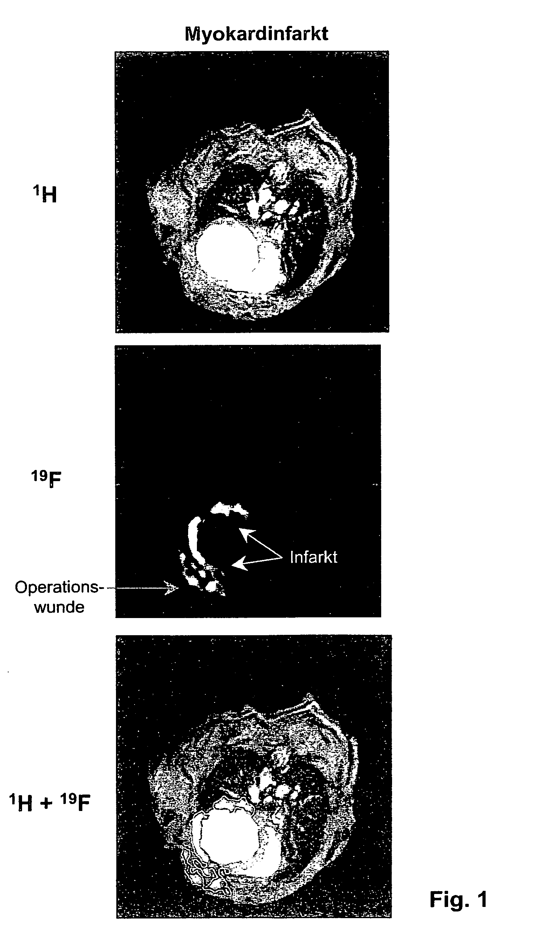

Anschließend wurden mit einem ¹H/¹⁹F-Resonator (Innendurchmesser 30 mm) bei einer Feldstärke von 9.4 Tesla (Bruker DRX Spektrometer) anatomisch übereinstimmende ¹H- und ¹⁹F-MR-Bilder aufgenommem (Field of view 3.3 cm² (Herz) bzw. 2.2 cm² (Hirn), ¹H: Cine-FLASH (Herz, EKG- und Respirationsgetriggert) bzw. RARE (Hirn, RARE-Faktor 16), Schichtdicke 1 mm, Matrix 256.256, ¹⁹F: RARE (RARE-Faktor 64), Schichtdicke 2 mm, Matrix 128·128. Durch die kombinierte Aufnahme von ¹H und ¹⁹F-Bildern konnte am Herzen eine Infiltration von PFCs in die Randbereiche des Infarkts nachgewiesen werden. Fig. 1 zeigt anatomisch übereinstimmende ¹H- + ¹⁹F-MR-Bilder aus dem Thorax einer Maus vier Tage nach Auslösen eines Myokardinfarkts (Fig. 1). Die Überlagerung zeigt deutlich die Anreicherung des Fluorsignals im Infarktbereich und in der Operationswunde.

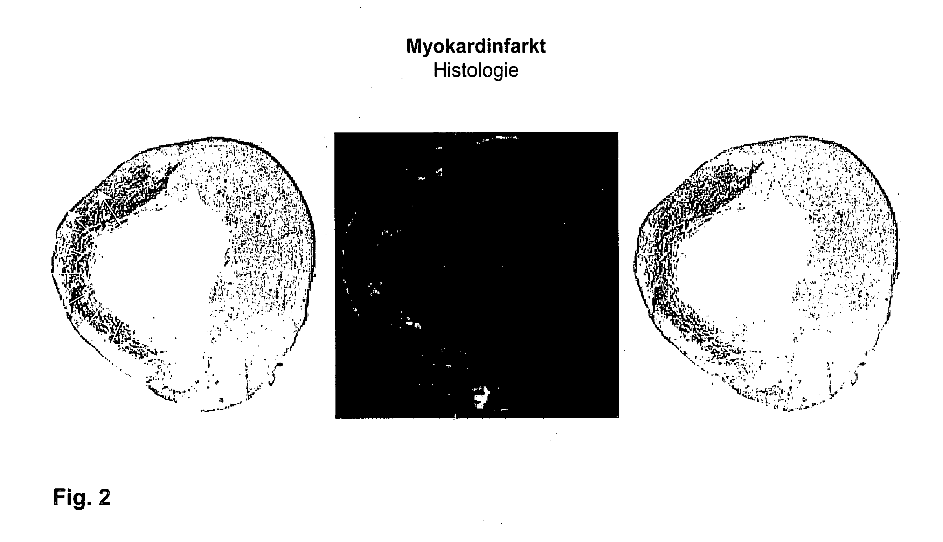

Histologische Hellfeld- und Rhodaminfluoreszenz-Übersichtsaufnahmen von Kryoschnitten (8 µm) der Mäuseherzen vier Tage nach Auslösen eines Myokardinfarkts (Fig. 2) bestätigen die *in vivo* Befunde. Die Überlagerung zeigt die Lokalisation der Rhodamin-markierten Perfluorcarbone im Randbereich des Infarkts.

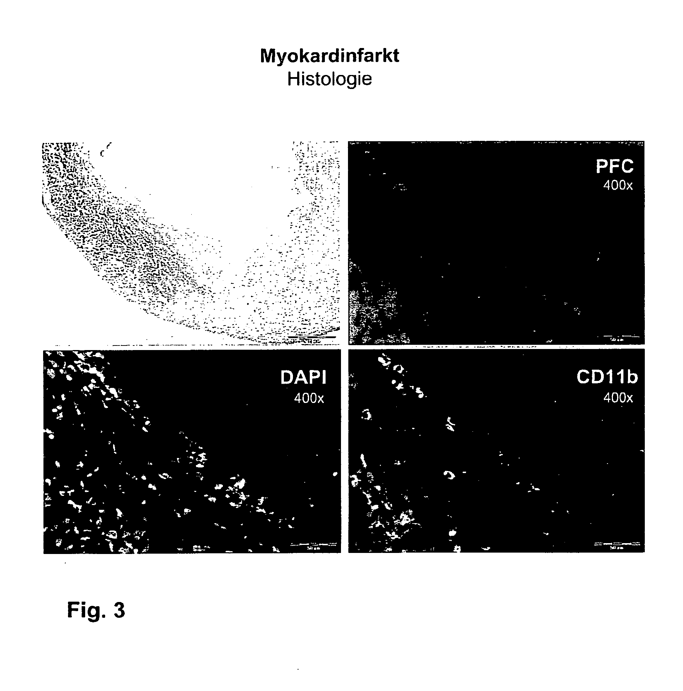

Die Kolokalisation von Makrophagen und PFCs konnte mittels CD11b-Anfärbung nachgewiesen werden. Fig. 3 zeigt Ausschnitte aus histologischen Hellfeld- und Fluoreszenz-Aufnahmen von Kryoschnitten (8 µm) eines Mäuseherzens vier Tage nach Auslösen eines Myokardinfarkts. Der Fluoreszenzvergleich zeigt deutlich die Kolokalisation von Rhodaminmarkierung (PFCs) und CD11b-positiven Zellen (Monozyten/ Makrophagen).

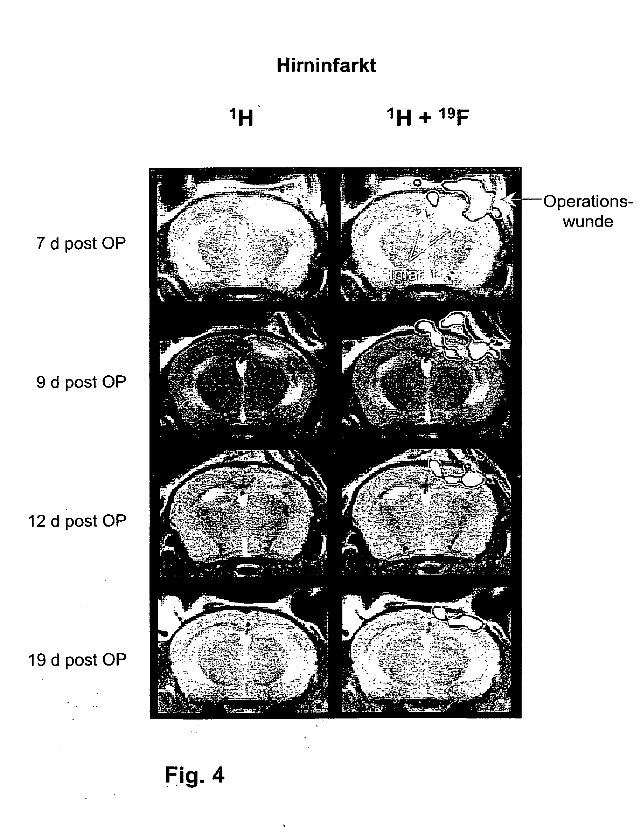

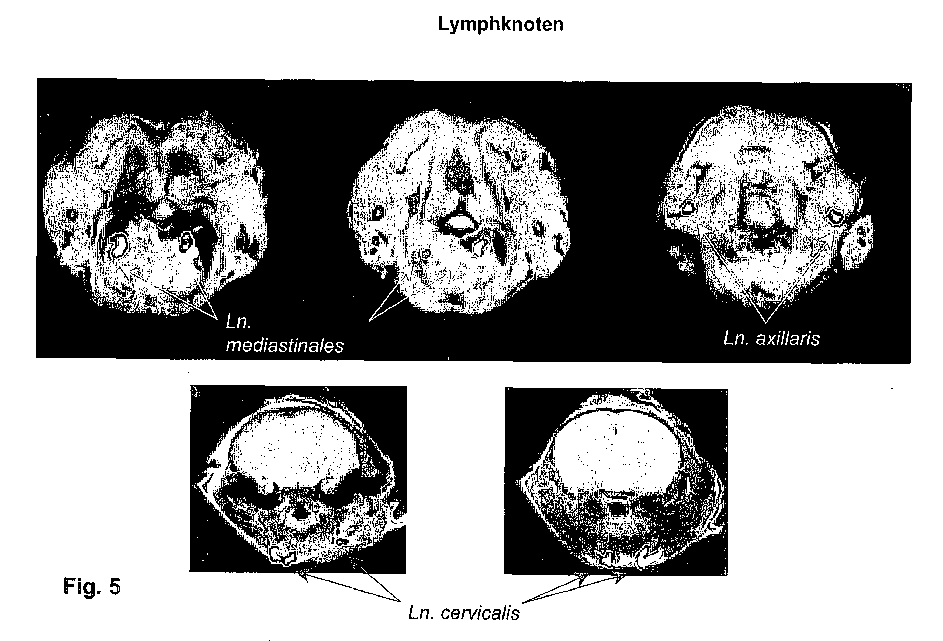

Ähnliche Ergebnisse wie am Herzen wurden auch nach Hirninfarkt durch Photothrombose beobachtet. Allerdings wurde das ¹⁹F-Signal hier erst nach 6-7 Tagen im Randbereich des Infarkts beobachtet (Fig. 4), was in Übereinstimmung mit dem aus anderen Studien bekanntem, verzögerten Eindringen der Makrophagen in diesem Modell ist. Auch hier ließen sich zusätzliche Signale in der Operationswunde und in den Lymphknoten beobachten (siehe weiter unten und Fig. 5). Fig. 4 zeigt zudem deutlich die Verschiebung der PFCs mit der Penumbra des nach einigen Tagen schrumpfenden Infarktareals.

Unabhängig von induzierten Verletzungen konnte in jedem Fall eine Anreicherung der PFCs in den Lymphknoten detektiert werden. Dies ist in Fig. 5 exemplarisch für die entsprechenden Lymphknoten im Bereich des oberen Brustkorbs sowie des Kopfes der Maus dargestellt. Es ist klar zu erkennen, daß sich die Perfluorcarbone in den Lymphknoten, die sich in diesen Arealen befinden, anreichern.

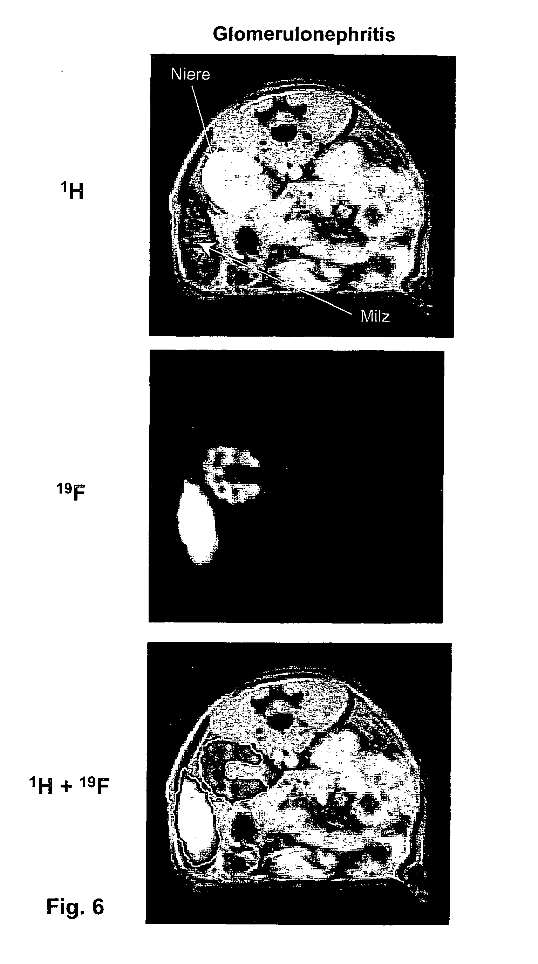

Die beschriebenen Befunde wurden in weiteren Krankheitsmodellen bestätigt. Die Abbildungen 6-9 zeigen, daß ¹⁹F-MR-Bildgebung nach Injektion von PFC-Emulsionen auch zur Darstellung von entzündlichen Nierenerkrankungen, zum Nachweis von Multipler Sklerose sowie Myokarditis und auch zur Visualisierung von inflammatorischen Gefäßerkrankungen eingesetzt werden kann. Im Folgenden sind die einzelnen Abbildung kurz erläutert:
Fig. 6 zeigt anatomisch übereinstimmende ¹H- + ¹⁹F-MR-Bilder aus dem Abdominalbereich einer Maus 4 Tage nach Induktion einer Glomerulonephritis. Die Überlagerung zeigt, daß sich das Fluorsignal in der Nierenrinde anreichert. Außerdem findet man erwartungsgemäß Signale in der Milz (retikuloendotheliales System).

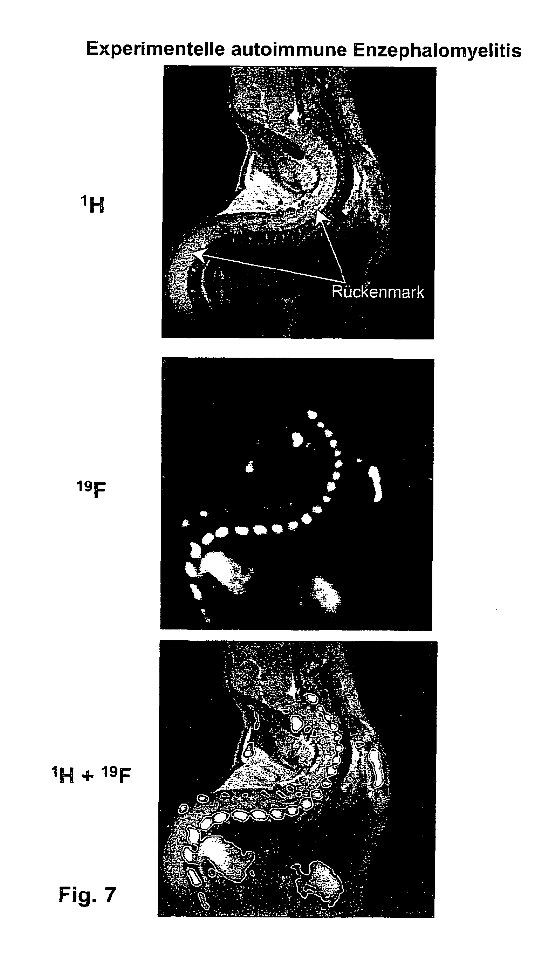

In Fig. 7 sind morphologisch korrespondierende ¹H- + ¹⁹F-MR-Bilder 7 Tage nach Auslösen einer experimentelle autoimmunen Enzephalomyelitis (Tiermodell für Multiple Sklerose) dargestellt, die eine Akkumulation der Perfluorcarbone im Rücken- bzw. Knochenmark demonstrieren. Außerdem findet man erwartungsgemäß Signale in der Leber (retikuloendotheliales System).

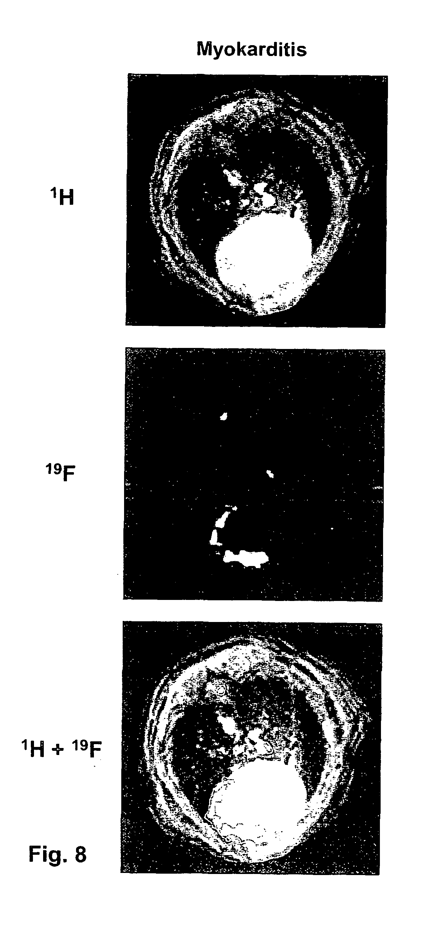

Fig. 8 zeigt anatomisch übereinstimmenden ¹H- + ¹⁹F-MR-Bilder, die 14 Tage nach Auslösen einer Myokarditis durch Injektion von Troponin I eine Anreicherung des Fluorsignals im entzündeten linken Ventrikel zeigen.

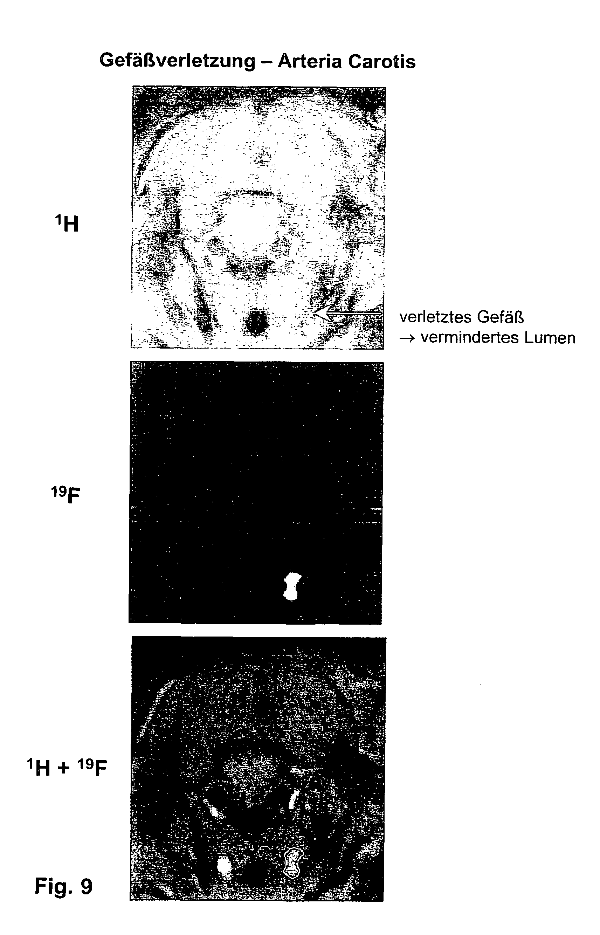

In Fig. 9 sind anatomisch korrespondierende ¹H- + ¹⁹F-MR-Bilder 7 Tage nach Denudation der rechten Arteria Carotis (Halsschlagader, Restenosemodell) abgebildet. Es läßt sich deutlich das Perfluorcarbonsignal um das verminderte Lumen im geschädigten Gefäß aufgrund gesteigerter Neointima-Bildung erkennen.

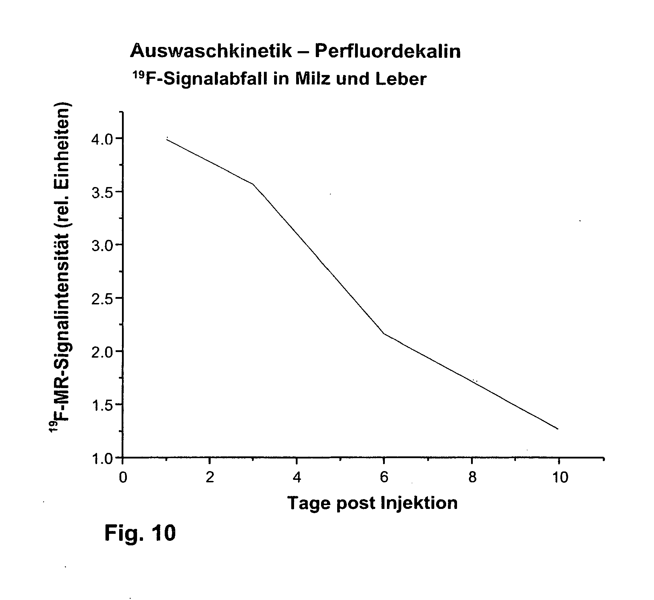

In weiteren Versuchen wurde demonstriert, daß sich durch Variation des verwendeten Perfluorcarbons eine Ausscheidung dieser Substanz innerhalb von wenigen Tagen erreichen läßt. Fig. 10 zeigt die Intensität des Fluorsignals in der Maus nach Injektion von 500 µl einer 40%igen Perfluordekalinemulsion. Nach ca. 10 Tagen ist das Perfluorcarbon nahezu vollständig aus dem Körper verschwunden.

Diese Ergebnisse zeigen, daß sich intravenös applizierte, emulgierte PFCs nach Phagozytose durch das Monozyten-Makrophagen-System in inflammatorischen Arealen sowie in Lymphknoten akkumulieren und mittels MRI mit hoher lokaler Auflösung nachweisen lassen. Die PFCs stellen somit ein "positives" Kontrastmittel für Entzündungsprozesse und das lymphatische System dar, das aufgrund eines fehlenden natürlichen ¹⁹F-Hintergrundes ein hohes Maß an Spezifität aufweist. Da PFCs bekanntlich nicht toxisch sind, sollte sich dieses Verfahren auch für die Anwendung am Menschen eignen.

## Patentansprüche

1. Verwendung eines in einem Vehikel vorliegenden Perfluorooktylbromids enthaltend mindestens ein 19F-Isotop zur Herstellung eines Medikaments für den diagnostischen Nachweis mittels eines bildgebenden Verfahrens von entzündlichen Vorgängen ausgewählt aus der Gruppe bestehend aus Entzündungsreaktionen im Randbereich von Infarkten, wie Herzinfarkt, Schlaganfall; Entzündungen von Organen wie Myokarditis, Encephalitis, Meningitis (Hirn und Rückenmarkshäute); Multiple Sklerose; Entzündungen des Magen-Darm-Traktes wie Morbus Crohn; Entzündungen der Gefäße wie Arteriosklerose insbesondere sog. "vulnerable plaques"; Detektion von Abzessen sowie Arthritis, wobei das bildgebende Verfahren auf Messung der magnetischen Kernresonanz des 19F-Isotops beruht, und das Vehikel ein Liposom mit einer Größe zwischen 100 und 200 nm ist.

2. Verwendung gemäß Anspruch 1, **dadurch gekennzeichnet, dass** das Vehikel ein unilamellares Liposom ist.

3. Verwendung nach Anspruch 1 oder 2, **dadurch gekennzeichnet, dass** das Vehikel Strukturen aufweist, die eine spezifische Markierung ausgewählter Ziele ermöglichen.

4. Verwendung nach Anspruch 3, **dadurch gekennzeichnet, dass** die Strukturen Moleküle sind, die eine Bindung der Vesikel an Epitope von Antigenen, Rezeptoren oder andere Proteine, eine Bindung an Nukleinsäuren und/oder Membranen ausgewählter Zellen, Gewebe- oder Organtypen, ermöglicht.

5. Verwendung nach Anspruch 3 oder 4, **dadurch gekennzeichnet, dass** die ausgewählten Zellen, Gewebe- oder Organtypen in einem pathologischen Zustand vorliegen.

## Claims

1. Use of a perfluorooctyl bromide present in a vehicle and comprising at least one 19F isotope for preparing a medicament for the diagnostic detection, by means of an imaging procedure, of inflammatory processes selected from the group consisting of inflammatory reactions peripheral to infarctions such as myocardial infarction, stroke; inflammations of organs, such as myocarditis, encephalitis, meningitis (of the meninx of the brain and the spinal meninx); multiple sclerosis; inflammations of the gastrointestinal tract, such as Crohn's disease; inflammations of the vessels, such as arterial sclerosis, in particular vulnerable plaques; detection of abscesses and also arthritis, where the imaging process is based on measuring the nuclear magnetic resonance of the ¹⁹F isotope and the vehicle is a liposome having a size from 100 to 200 nm.

2. Use according to Claim 1, **characterized in that** the vehicle is a unilamellar liposome.

3. Use according to Claim 1 or 2, **characterized in that** the vehicle has structures allowing a specific labelling of selected targets.

4. Use according to Claim 3, **characterized in that** the structures are molecules allowing the vesicles to bind to epitopes of antigens, receptors or other proteins, to nucleic acids and/or membranes of selected cells, tissue or organ types.

5. Use according to Claim 3 or 4, **characterized in that** the selected cells, tissue or organ types are present in a pathologic state.

## Revendications

1. Utilisation d'un bromure de perfluoro-octyle, contenant au moins un isotope ¹⁹F, présent dans un véhicule, pour la fabrication d'un médicament destiné à la détection diagnostique par un procédé d'imagerie de processus inflammatoires choisis dans le groupe consistant en des réactions inflammatoires dans le secteur englobant les infarctus, tels que l'infarctus du myocarde, l'accident vasculaire cérébral ; les inflammations d'organes telles que la myocardite, l'encéphalite, la méningite (méninges cérébrale et médullaire) ; la sclérose en plaques ; les inflammations du tractus gastro-intestinal telles que la maladie de Crohn ; les inflammations des vaisseaux telles que l'artériosclérose en particulier les dénommées « vulnerable plaques » ; la détection d'abcès ainsi que d'arthrite, le procédé d'imagerie reposant sur la mesure de la résonance magnétique nucléaire de l'isotope ¹⁹F, et le véhicule étant un liposome ayant une taille comprise entre 100 et 200 nm.

2. Utilisation selon la revendication 1, **caractérisée en ce que** le véhicule est un liposome unilamellaire.

3. Utilisation selon la revendication 1 ou 2, **caractérisée en ce que** le véhicule présente des structures qui permettent un marquage spécifique de cellules choisies.

4. Utilisation selon la revendication 3, **caractérisée en ce que** les structures sont des molécules qui permettent une liaison des vésicules à des épitopes d'antigènes, des récepteurs ou d'autres protéines, une liaison à des acides nucléiques et/ou à des membranes de cellules, types de tissus ou d'organes, choisis.

5. Utilisation selon la revendication 3 ou 4, **caractérisée en ce que** les cellules, types de tissus ou d'organes, choisis, se trouvent en un état pathologique.
